(19) Europäisches Patentamt / European Patent Office / Office européen des brevets

(11) **EP 3 346 014 B1**

(12) **EUROPEAN PATENT SPECIFICATION**

(45) Date of publication and mention
of the grant of the patent:
**08.11.2023  Bulletin 2023/45**

(51) International Patent Classification (IPC):
**G01N 33/50** *(2006.01)*    **C12N 15/09** *(2006.01)*
**C12Q 1/6886** *(2018.01)*

(21) Application number: **16841968.7**

(22) Date of filing: **01.09.2016**

(52) Cooperative Patent Classification (CPC):
**C12Q 1/6886; C12Q 1/6827; G01N 33/50;**
C12N 15/09; C12Q 2600/154

(86) International application number:
**PCT/JP2016/075667**

(87) International publication number:
**WO 2017/038939 (09.03.2017 Gazette 2017/10)**

(54) **METHOD FOR DETECTING PRECANCEROUS ORAL LESION**

VERFAHREN ZUM NACHWEIS PRÄKANZERÖSER ORALER LÄSIONEN

PROCÉDÉ DE DÉTECTION DE LÉSION BUCCALE PRÉCANCÉREUSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **01.09.2015   JP 2015172327**

(43) Date of publication of application:
**11.07.2018  Bulletin 2018/28**

(73) Proprietor: **Tohoku University
Sendai-shi, Miyagi 980-8577 (JP)**

(72) Inventor: **HAMADA, Tomofumi
Kagoshima-shi
Kagoshima 890-8580 (JP)**

(74) Representative: **J A Kemp LLP
80 Turnmill Street
London EC1M 5QU (GB)**

(56) References cited:
**WO-A1-2010/001933      WO-A1-2013/074793
WO-A1-2014/032205      WO-A2-2006/079014
WO-A2-2008/036333      JP-A- 2010 161 984**

- **G. S. ASOKAN: "Promoter Hypermethylation
  Profile of Tumour Suppressor Genes in Oral
  Leukoplakia and Oral Squamous Cell
  Carcinoma", JOURNAL OF CLINICAL AND
  DIAGNOSTIC RESEARCH, 20 October 2014
  (2014-10-20), XP055531007, ISSN: 2249-782X,
  DOI: 10.7860/JCDR/2014/9251.4949**
- **TAKANOBU KUSUMOTO ET AL:
  "Comprehensive Epigenetic Analysis Using Oral
  Rinse Samples: A Pilot Study", JOURNAL OF
  ORAL AND MAXILLOFACIAL SURGERY., vol. 70,
  no. 6, 1 June 2012 (2012-06-01), pages 1486-1494,
  XP055531019, US ISSN: 0278-2391, DOI:
  10.1016/j.joms.2011.04.021**
- **K. M. PATTANI ET AL: "Endothelin Receptor Type
  B Gene Promoter Hypermethylation in Salivary
  Rinses Is Independently Associated with Risk of
  Oral Cavity Cancer and Premalignancy",
  CANCER PREVENTION RESEARCH, vol. 3, no. 9,
  26 August 2010 (2010-08-26) , pages 1093-1103,
  XP055530965, United States ISSN: 1940-6207,
  DOI: 10.1158/1940-6207.CAPR-10-0115**
- **J. SCHUSSEL ET AL: "EDNRB and DCC Salivary
  Rinse Hypermethylation Has a Similar
  Performance as Expert Clinical Examination in
  Discrimination of Oral Cancer/Dysplasia versus
  Benign Lesions", CLINICAL CANCER
  RESEARCH, vol. 19, no. 12, 1 May 2013
  (2013-05-01), pages 3268-3275, XP055530970, US
  ISSN: 1078-0432, DOI:
  10.1158/1078-0432.CCR-12-3496**

- **KENJI OKAMI et al.: "Daekichu Gan Saibo no p16 Idenshi Ijo Methyl-ka o Bunshi Seibutsugakuteki Shindan ni Oyo Dekiru ka", Proceedings of the Japanese Cancer Association, vol. 63, 25 August 2004 (2004-08-25), page 177, XP009509280,**
- **TRAN T. N. et al.: "Frequent promoter hypermethylation of RASSF1A, p16INK4A but infrequent allelic loss other than 9p21 in betel-associated oral carcinoma.", J. ORAL BIOSCI., vol. 46, no. 5 , page 387, XP009508847,**
- **KEIZO KATO et al.: "Koku Ryoiki ni Okeru p16·MGMT Idenshi no Promoter Ryoiki no Methyl-ka Ijo", Proceedings of the Japanese Cancer Association, vol. 63, 25 August 2004 (2004-08-25), page 177, XP009509435,**
- **CHUANG, C.Y. et al.: "PL-9-3 Methylation status of PRKCB, RARbeta and p16 genes promoter and global methylation in oral cancer", Annual Meeting of Japanese Society of Medical Oncology, vol. 13, 16 July 2015 (2015-07-16), pages 1-3, XP009509564,**

Remarks:

The complete document including Reference Table(s) and the Sequence Listing(s) can be downloaded from the EPO website

**Description**

Technical Field

The present invention relates to a method for detecting an oral precancerous lesion. More specifically, the present invention relates to a method for noninvasively detecting an oral precancerous lesion by analyzing DNA contained in gargled fluid.

Background Art

The oral cavity is an organ that has important functions such as mastication, swallowing, and articulation, which are essential for human living and life. However, in general, the level of knowledge of oral cancer is low. Since there are few initial subjective symptoms of oral cancer and there are many mucosal diseases presenting with similar clinical symptoms, early detection of oral cancer has not been achieved to a sufficient extent. The 5-year survival rate of oral cancer is about 65%.

For oral cancer, there is no established noninvasive screening method such as fecal occult blood test for large bowel cancer, X-ray examination for gastric cancer, mammography for breast cancer, or the like. Oral cancer screening including detection of oral precancerous lesions requires examination by specialists. At present, large-scale screening is impossible in practice. Although there is a screening test involving staining with a dye such as toluidine blue, diagnostic accuracy is not always satisfactory.

Meanwhile, leukoplakia is a precancerous lesion of oral cancer, which is a white hyperkeratotic lesion found on buccal mucosa, tongue, gum, or the like at a relatively high frequency. Although known causes of leukoplakia include irritation due to smoking or alcohol, mechanical irritation due to denture, lack of vitamins, and the like. There are many leukoplakia cases with unknown causes. The lesion sometimes accompanies a bulging or aching. However, in the case of a non-bulging or non-aching lesion, the development of the lesion is often not noticed. Nevertheless, a lesion formed on the tongue is highly likely to become malignant, and is considered to be a representative precancerous lesion. Resection is the most reliable treatment for leukoplakia. However, if a lesion spreads widely, resection may cause functional disorder. Accordingly, it is considered appropriate to detect the lesion in the early phase and determine the treatment strategy while monitoring the subject.

As a method for detecting oral cancer, a method for detecting oral disease metabolites or transcriptome pattern in saliva is suggested (Patent Literatures 1 and 2).

It has been reported that DNA methylation, specifically, a reaction of adding a methyl group to a carbon atom at 5-positoin of a pyrimidine ring of cytosine, for example, is involved in epigenetics and associated with carcinogesis. The present inventors previously found and reported the group of genes showing abnormal methylation specific to oral cancer (Non Patent Literatures 1 and 2). The inventors reported the methods disclosed in Non Patent Literatures 1 and 2, by which noninvasive detection of oral squamous cell carcinoma can be achieved by detecting abnormal methylation of specific tumor-related genes contained in gargled fluid of a subject.

However, there have been no reports on precancerous lesion detection methods.

Citation List

Patent Literature

    Patent Literature 1: JP Patent Publication (Kokai) No. 2011-229532 A
    Patent Literature 2: JP Patent Publication (Kohyo) No. 2013-505428 A

Non Patent Literature

    Non Patent Literature 1: J. Oral. Maxillofac Surg., Vol. 70, Issue 6, pp. 1486-1494, August 6, 2011
    Non Patent Literature 2: Cancer, September 1, 2012, DOI: 10.1002/cncr.27417

Summary of Invention

Technical Problem

As described above, the oral cavity is an organ having important functions, and it is necessary to immediately take a measure in the case where any abnormality is found. In addition, since there is currently no established therapy for drastically improving the survival rate of oral cancer patients, early detection and early treatment are the most certain and practical measures. From the viewpoints of better prognosis and functional preservation, it is very important to detect oral precancerous lesions early on.

Solution to Problem

In view of the above problems, the present inventors examined how to establish a noninvasive method, which enables early detection of precancerous lesions of leukoplakia and the like, which may induce oral cancer, by large-scale screening.

As a result, the present inventors succeeded in identifying cancer suppressor genes showing specific abnormal methylation using gargled fluid as a specimen and establishing an oral precancerous lesion detection system with high accuracy using such genes.

The invention is defined in the appended set of claims. Specifically, the following is provided according to the present invention.

1. A method for obtaining data for diagnosis of an oral precancerous lesion in a subject by detecting DNA methylation in gargled fluid collected from the subject, wherein the DNA is DNA in a promoter region of one, two or three genes selected from the following group of genes:

RASSF1 (Ras association domain family member 1, NM_170714.1),
CD44 (NM_001001391.1), and
BRCA2 (breast cancer susceptibility gene 2, NM_000059.3).

2. The method according to 1 above, which comprises comparing detected DNA methylation and DNA methylation in a control sample.

3. The method according to 1 or 2 above, wherein DNA methylation in the promoter regions of RASSF1 and BRCA2 genes is detected.

4. The method according to 1 or 2 above, wherein DNA methylation in the promoter regions of DAPK1 (death-associated protein kinase 1, NM_004938.2), and FHIT (fragile histidine triad gene, NM_002012.2) genes is further detected.

5. The method according to 1 or 2 above, wherein DNA methylation is detectable using a probe having any of the nucleotide sequences shown in SEQ ID NOS: 5 to 8, 19, and 20.

6. The method according to any one of 1 to 5 above, wherein the oral precancerous lesion is leukoplakia.

7. Use of primers or at least one probe for detecting abnormal methylation of DNA selected from DNAs of the promoter regions of RASSF1, CD44 and BRCA2 genes, for detecting an oral precancerous lesion in gargled fluid collected from a subject.

8. The use of at least one probe according to 7 above, wherein the at least one probe has any of the nucleotide sequences shown in SEQ ID NOS: 5 to 7, 8, 19, and 20.

9. The use of primers or at least one probe according to 7 or 8 above, wherein the oral precancerous lesion is leukoplakia.

The present description includes part or all of the contents as disclosed in the description and/or drawings of Japanese Patent Application No. 2015-172327, which is a priority document of the present application.

Advantageous Effects of Invention

According to the present invention, oral precancerous lesions of leukoplakia and the like can be noninvasively detected using gargled fluid in a convenient manner. Since DNAs are relatively stable, compared with proteins and RNAs, DNAs are excellent in terms of storage and transportation. In addition, an abnormality in DNA methylation is found in an early-stage lesion or before the development of a lesion. Therefore, abnormal DNA methylation that has been discovered by the present inventors is a useful marker for early detection of oral cancer and evaluation of the carcinogenic risk.

Brief Description of Drawings

[Figure 1] Figure 1 shows distribution of DNA abnormal methylation in the promoter regions of 15 types of genes, which were compared between the healthy group and the leukoplakia group. The vertical axis indicates the average percentage of methylation in each gene promoter region in each group. High-level methylation in the leukoplakia group was observed in every gene with a statistical significance.

[Figure 2] Figure 2 shows ROC curves for accuracy of leukoplakia detection regarding DNA methylation in the promoter regions of 7 genes having particularly high AUC values in the group of genes listed in Table 2. In all cases, AUC > 0.85 was achieved, indicating favorable detection rates.

[Figure 3] Figure 3 shows distribution of abnormal methylation in the promoter regions of 7 genes in the leukoplakia group and the healthy group. Each cell denotes one subject in the leukoplakia group or the healthy group, a white cell indicates the nonoccurrence of abnormal methylation, and a black cell indicates the occurrence of abnormal methylation. It was confirmed that there are clear differences in the distribution between the leukoplakia group and the healthy group.

[Figure 4] Figure 4A shows the result of logistic analysis for methylation in the promoter regions of RASSF1, FHIT, DAPK1, CD44, and BRCA2 genes.

Figure 4B shows an ROC curve based on the results for RASSF1, FHIT, DAPK1, CD44, and BRCA2 genes. Each result indicates favorable detection accuracy.

Description of Embodiments

The present invention provides a method for obtaining data for diagnosing an oral precancerous lesion in a subject by detecting DNA methylation in a specific gene contained in gargled fluid collected from the subject. Since "gargled fluid" contains oral exfoliated mucosal cells in the entire oral cavity and it is easy to collect and treat such fluid for detection, gargled fluid is particularly preferable for noninvasive diagnosis. According to the present invention, subjects are human subjects, which include patients who may have oral abnormalities and subjects who may be targets of wide-scale screening such as health examination.

The term "gargled fluid" used herein refers to a liquid sample obtained after a subject gargles with an adequate amount of water or an aqueous solution (gargling). Examples of an aqueous solution include, but are not particularly limited, physiological saline, distilled water, and usual tap water.

The way of gargling is not particularly limited. However, in view of secure DNA sampling, burden on a subject, and the like, it is preferable to put physiological saline or the like into the mouth in an amount of, for example, about 10 to 50 mL and preferably about 20 to 30 mL and gargle for 20 to 90 seconds and preferably 30 to 60 seconds.

Examples of an "oral precancerous lesion" described herein include, but are not limited to, lesions of leukoplakia, erythroplakia, and lichen planus.

According to the method of the present disclosure, DNA, which is a target of DNA methylation detection that is preferable for detection of an oral precancerous lesion, is at least one type of DNA selected from DNAs in the promoter regions of the following group of genes:

RASSF1 (Ras association domain family member 1),
DAPK1 (death-associated protein kinase 1),
CD44,
BRCA2 (breast cancer susceptibility gene 2),
FHIT (fragile histidine triad gene),
CDKN2A (cyclin-dependent kinase inhibitor 2A),
HIC1 (hypermethylated in cancer 1),
CASP8 (caspase 8),
RARβ (retinoic acid receptor beta),
CDKN2B (cyclin-dependent kinase inhibitor 2B),
CHFR (checkpoint with forkhead and ring finger domains, E3 ubiquitin protein ligase),
ATM (ataxia telangiectasia mutated),
CDKN1B (cyclin-dependent kinase inhibitor 1B),
BRCA1 (breast cancer susceptibility gene 1), and
CADM1 (cell adhesion molecule 1).

Table 1 lists gene names, symbols, chromosomal loci, and NCBI accession numbers for the above-described

5

genes.

[Table 1]

| Symbol | Gene Name | Position | NCBI Accession No. |
|---|---|---|---|
| CDKN2A | Cyclin-dependent kinase inhibitor 2A | 9p21.3 | NM_058195.3 |
| DAPK1 | Death-associated protein kinase 1 | 9q21.31 | NM_004938.2 |
| CD44 | CD44 molecule | 11p13 | NM_001001391.1 |
| RASSF1 | Ras association domain family member 1 | 3p21.31 | NM_170714.1 |
| ATM | Ataxia telangiectasia mutated | 11q22.3 | NM_000051.3 |
| CADM1 | Cell adhesion molecule | 11q23.3 | NM_001098517.1 |
| CHFR | Checkpoint with forkhead and ring finger domains, E3 ubiquitin protein ligase | 12q24.33 | NM_001161344.1 |
| FHIT | Fragile histidine triad gene | 3p14.2 | NM_002012.2 |
| CDKN1B | Cyclin-dependent kinase inhibitor 1B | 12p13.1 | NM_004064.3 |
| BRCA2 | Breast cancer susceptibility gene 2 | 13q12.3 | NM_000059.3 |
| HIC1 | Hypermethylated in cancer 1 | 17p13.3 | NM_006497.3 |
| BRCA1 | Breast cancer susceptibility gene 1 | 17q21.31 | NM_007294.3 |
| CASP8 | Caspase 8 | 2q33.1 | NM_001080125 |
| RARB | Retinoic acid receptor beta | 3p24.2 | NM_000965.3 |
| CDKN2B | Cyclin-dependent kinase inhibitor 2B | 9p21.3 | NM_078487.2 |

In the present disclosure, the occurrence or nonoccurrence of DNA methylation in the promoter region of at least one gene selected from the subject-derived genes listed in Table 1 is detected, thereby obtaining data for diagnosing an oral precancerous lesion in a subject. It is possible to determine that the subject is likely to have an oral precancerous lesion in the case of the occurrence of DNA methylation, or that the subject is unlikely to have such lesion in the case of the nonoccurrence of DNA methylation. Data used for diagnosis may be data of 2 or more, 3 or more, 4 or more, 5 or more, 10 or more, or all of the above genes.

One aspect of the present disclosure concerns the above method, which comprises detecting methylation of at least one DNA selected from DNAs in the promoter regions of RASSF1, DAPK1, CD44, BRCA2, FHIT, CDKN2A, and HIC1 genes.

Another aspect of the present disclosure concerns the above method, which comprises detecting methylation of at least one DNA selected from DNAs in the promoter regions of RASSF1, DAPK1, CD44, and BRCA2 genes.

Yet another aspect of the present disclosure concerns the above method, which comprises detecting methylation of at least one DNA selected from DNAs in the promoter regions of RASSF1, CD44, BRCA2, DAPK1, and FHIT genes.

The present disclosure also provides a method for diagnosing the presence of an oral precancerous lesion or a probability of having an oral precancerous lesion for a subject by detecting DNA methylation in a specific gene in gargled fluid collected from the subject. According to the present method, it is possible to diagnose whether or not a subject has an oral precancerous lesion of leukoplakia, erythroplakia, lichen planus, or the like, or whether or not a subject has a high risk of acquiring such disease. It is also possible to diagnose a probability of acquiring oral cancer according to the present method.

More specifically, the present method comprises:

detecting the occurrence (presence) or nonoccurrence (absence) of methylation in at least one DNA selected from DNAs in the promoter regions of the following 15 genes: RASSF1, DAPK1, CD44, BRCA2, FHIT, CDKN2A, HIC1, CASP8, RARβ, CDKN2B, CHFR, ATM, CDKN1B, BRCA1, and CADM1, and
comparing detected DNA methylation and DNA methylation in a control sample.

One aspect of the present method comprises detecting methylation of at least one type of DNA selected from DNAs in the promoter regions of RASSF1, DAPK1, CD44, BRCA2, FHIT, CDKN2A, and HIC1 genes.

Another aspect of the present method comprises detecting methylation of at least one type of DNA selected from DNAs in the promoter regions of RASSF1, DAPK1, CD44, and BRCA2 genes.

Yet another aspect of the present method comprises detecting methylation of at least one type of DNA selected from DNAs in the promoter regions of RASSF1, CD44, BRCA2, DAPK1, and FHIT genes.

Currently, various databases have been constructed, and DNA sequences of genes and the like in the human genome are readily available by conducting search based on gene names, database accession numbers (reference numbers), or the like. The object of the present invention is to detect methylation in a target DNA sequence by a method capable of discriminating a sequence including a methylated base and an unmethylated sequence.

For example, when a sample is treated with bisulfite in advance, unmethylated cytosine is converted into uracil, which generates a sequence that differs from a sequence including methylated cytosine. Based on this method, two sets of primers, by which a sequence including methylated cytosine and a sequence including unmethylated cytosine can be separately amplified, are designed, and the occurrence or nonoccurrence of amplification or the sequence of amplified DNA can be determined by PCR reactions. Examples of a commercially available kit that can be used for this method include EpiScope MSP Kits (Takara Bio Inc.). The primer length is not particularly limited. However, an amplification product having a length of 80 to 500 bases, which includes bases that might be methylated, can be obtained using primers having lengths of, for example, 20 to 45 bases.

Alternatively, it is also possible to employ the methylation specific-multiplex ligation probe amplification (MS-MLPA) method, which comprises setting a probe capable of hybridizing with a target region such that a PCR product can be obtained only when hybridization of the probe occurs. Such probe can be designed based on the target base sequence. For example, a commercially available MS-MLPA kit (MRC-Holland) includes probes capable of detecting abnormal methylation in the promoter regions of 26 types of cancer suppressor genes. With the use of this kit, it is possible to allow a 5'-end probe and a 3'-end probe to hybridize with a target sequence, conduct ligation, and carry out amplification through a PCR reaction using universal primers linked to both ends of the probes. There is a probability that DNA methylation have occurred in the target sequence. Therefore, it is possible to cleave, for example, an unmethylated hybridization product with the use of methylation-sensitive restriction enzyme during ligation described above. Accordingly, only when methylation has occurred, an amplification product can be obtained. There are various enzymes known as methylation-sensitive restriction enzymes, and examples thereof include AccII, HhaI, HapII, and HaeIII. These enzymes can be obtained from, for example, Takara Bio Inc.

The probe length is not particularly limited. However, for example, a probe having a length of 80 to 500 bases that is capable of hybridizing with a region including a base site, at which methylation might occur, can be used.

Examples of probes that can be preferably used in the method of the present disclosure include, but are not limited to, probes having the nucleotide sequences shown in SEQ ID NOS: 1 to 30. Therefore, in one aspect, the method of the present disclosure involves detecting DNA methylation with the use of probes having the nucleotide sequences shown in SEQ ID NOS: 1 to 30. In one embodiment, the method of the present invention involves detecting DNA methylation using at least one probe having any of the nucleotide sequences shown in SEQ ID NOS: 5 to 7, 8, 19, and 20.

After PCR reaction, it is possible to detect the presence or absence of each amplification product by conducting, for example, capillary electrophoresis.

Therefore, the method of the present invention involves, but is not particularly limited to, judging methylation of specific DNA described above based on the occurrence or nonoccurrence of cleavage of the DNA with a methylation-sensitive restriction enzyme such as HhaI as an index. HhaI is a *Haemophilus haemolyticus* (ATCC 10014)-derived restriction enzyme. In the case where DNA is cleaved with HhaI, it is possible to judge that DNA methylation has not occurred. In the case where DNA is not cleaved with HhaI, it is possible to judge that methylation of the DNA has occurred. Note that the promoter regions of the above-described genes listed in Table 1 includes a GCGC sequence, which is a HhaI cleavage site, and a probe can be prepared to include the sequence.

Detection of DNA methylation is not limited to the method described above, and any means used in the art may be employed.

The method of the present invention provides data for detecting an abnormality in DNA methylation in the promoter regions of the specific genes described above, and judging whether or not a subject has an oral precancerous lesion based on detection results. The term "abnormality in methylation" or "abnormal methylation" used herein means methylation that is not detected in a normal sample (control sample) from a healthy individual.

In the case of a cancer suppressor gene, the gene must be expressed in the normal state. Methylation causes inhibition of the expression of a cancer suppressor gene, it results in carcinogenesis. It is therefore considered that the promoter site of a cancer suppressor gene in a healthy individual is not methylated. However, methylation may occur in the normal state, and potential methylation, which is not related to a disease, may occur even in healthy individuals. In such cases, methylation occurring at the site is not judged as "abnormal." Therefore, it is preferable to compare detected DNA methylation with DNA methylation in a control sample.

Accordingly, the method of the present invention may include a step of detecting methylation of DNAs in the promoter regions of the above genes contained in gargled fluid collected from a subject and a step of comparing detected methylation with (the occurrence or nonoccurrence of) methylation of DNA in the same corresponding region in a control sample. In the case where methylation that differs from methylation in a control sample is confirmed, it suggests the possibility that there may be an oral precancerous lesion. However, since data of methylation in a normal sample (control sample) can be obtained in advance, it will be understood that it is not necessary to obtain information on methylation in a control sample at the same time for diagnosing an oral precancerous lesion for a subject.

DNAs for which the present inventors found significant methylation in an oral precancerous lesion, were identified using gargled fluid obtained from patients of leukoplakia, a representative oral precancerous lesion, and gargled fluid obtained from healthy individuals. A significant increase in the level of methylation as compared with that in healthy individuals can be confirmed using a statistical technique that is usually used in the art. Examples of such technique include, but are not limited to, t-test, Mann-Whitney U test, ROC curve (receiver operating characteristic curve) creation, and logistic regression analysis.

In the case where the level of methylation of DNAs specified above in gargled fluid collected from a subject increases significantly as compared with the level of methylation in a control sample, it suggests that the subject has an oral precancerous lesion or has a risk of developing an oral precancerous lesion. Note that the "significantly" corresponds to a significance level of not more than 5% and preferably not more than 1%, at which a null hypothesis is rejected in various statistical techniques.

Also disclosed but not claimed is a kit for detecting an oral precancerous lesion, which includes primers or probes for detecting abnormal methylation of DNA selected from DNAs in the promoter regions of RASSF1, DAPK1, CD44, BRCA2, FHIT, CDKN2A, HIC1, CASP8, RARβ, CDKN2B, CHFR, ATM, CDKN1B, BRCA1, and CADM1 genes. It is possible to use only specific primers or probes in the kit depending on DNAs to be detected. A kit only comprising such specific primers or probes is disclosed but not claimed.

For example, in order to detect DNA methylation in primer regions of RASSF1, DAPK1, CD44, and BRCA2 genes, probes having the nucleotide sequences shown in SEQ ID NOS: 3 to 8, 19, and 20 can be included in a kit.

It is preferable that such kit includes primers or probes particularly suitable for diagnosis of oral precancerous lesions. Therefore, the kit of the present disclosure may include, for example, primers or probes for detecting abnormal methylation of at least one DNA selected from DNAs in the promoter regions of RASSF1, DAPK1, CD44, BRCA2, FHIT, CDKN2A, and HIC1 genes. In addition, the kit of the present disclosure may include primers or probes for detecting abnormal methylation of at least one DNA selected from DNAs in the promoter regions of RASSF1, CD44, and BRCA2 genes. Further, the kit of the present disclosure may include primers or probes for detecting abnormal methylation of at least one DNA selected from DNAs in the promoter regions of RASSF1, CD44, BRCA2, DAPK1, and FHIT genes.

Examples of probes that can be included in the kit of the present disclosure include one or more probes having the nucleotide sequences shown in SEQ ID NOS: 1 to 30.

The kit may include an aqueous solution used for gargled fluid such as physiological saline, various reagents for, DNA extraction, hybridization, ligation, and PCR reaction, and restriction enzymes, if appropriate.

Examples

Subjects were leukoplakia patients who were clinically suspected to have leukoplakia and given a histological definite diagnosis (n = 19) and 55 healthy individuals as a control group who were found to have no abnormalities upon oral exploration.

Each subject was instructed to gargle with 20 ml of physiological saline for 30 seconds, and the full amount of gargled fluid was collected. The obtained sample in an amount of 5 ml was centrifuged at 2,000 rpm for 5 minutes (KUBOTA 6800 (KUBOTA Corporation)), washed with physiological saline, and centrifuged again at 2,000 rpm for 5 minutes. This operation was repeated twice, thereby obtaining a pellet. The pellet was stored at -80°C before use.

DNA was extracted using DNeasy Blood and Tissue Kits (Qiagen, Valencia, CA), and MS-PLPA was performed using MS-MLPA kit (MRC-Holland) in accordance with an ordinary method. As a probe set, probes corresponding to 26 types of genes each having an HhaI cleavage site, which are ME001-C1 Tumor suppressor-1 included in the kit, were used. Two types of probes were used for one gene, and either one or both of the probes have one or two HhaI cleavage sites. Cleavage with HhaI (Promega Corporation) was conducted together with hybridization and ligation, followed by PCR. Fragment analysis was performed using 3130 Genetic Analyser (ABI Inc.). After calculation of the peak area using Gene Mapper (v4.1, ABI Inc.), statistical processing was conducted.

[Distribution of Abnormal DNA Methylation and Comparison of Leukoplakia Group and Healthy Group]

The methylation status of 26 types of cancer suppressor genes contained in the MS-MLPA kit was examined. The methylation levels of individual quantitatively determined genes were compared and examined by the t-test or Mann-Whitney U test.

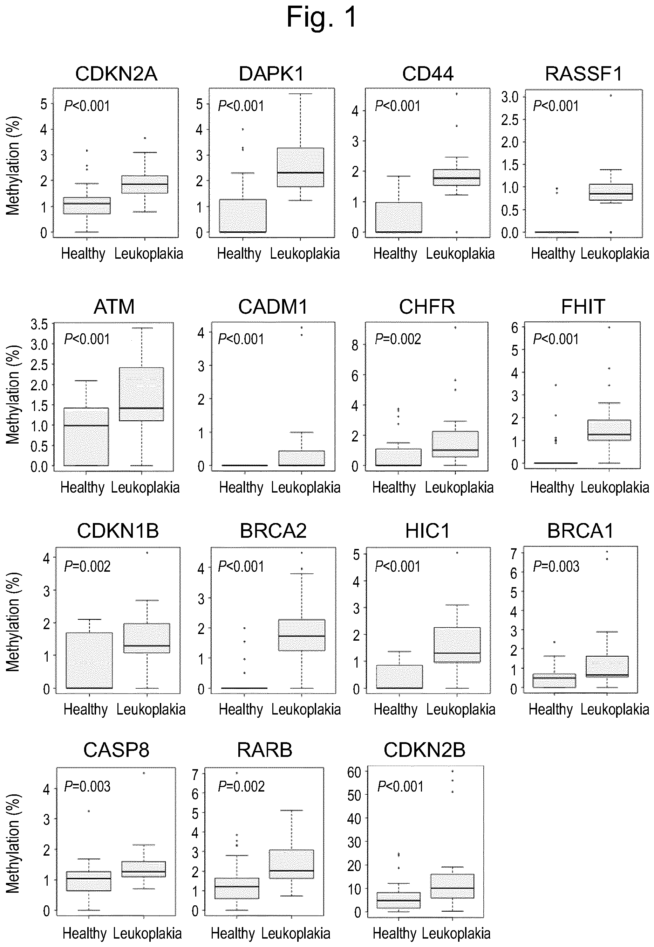

Table 2 lists symbols representing 15 types of genes which were found to have an increase in abnormal methylation in the promoter region in the leukoplakia group as compared with the healthy group, and the sequence identification numbers of probes used for detection. In addition, distribution of abnormal DNA methylation in these gene promoter regions were compared between the healthy group and the leukoplakia group. The results are boxplotted in Figure 1.

[Table 2]

| Symbol | Probe | Position | Initiation Position | Termination Position |
|---|---|---|---|---|
| CDKN2A | SEQ ID NOS: 1 and 2 | 9p21.3 | 21985276 | 21985349 |
| DAPK1 | SEQ ID NOS: 3 and 4 | 9q21.31 | 89303075 | 89303130 |
| CD44 | SEQ ID NOS: 5 and 6 | 11p13 | 35117389 | 35117448 |
| RASSF1 | SEQ ID NOS: 7 and 8 | 3p21.31 | 50353347 | 50353403 |
| ATM | SEQ ID NOS: 9 and 10 | 11q22.3 | 107599044 | 107599105 |
| CADM1 | SEQ ID NOS: 11 and 12 | 11q23.3 | 114880585 | 114880649 |
| CHFR | SEQ ID NOS: 13 and 14 | 12q24.33 | 131974372 | 131974433 |
| FHIT | SEQ ID NOS: 15 and 16 | 3p14.2 | 61211918 | 61211970 |
| CDKN1B | SEQ ID NOS: 17 and 18 | 12p13.1 | 12761863 | 12761918 |
| BRCA2 | SEQ ID NOS: 19 and 20 | 13q12.3 | 31787722 | 31787786 |
| HIC1 | SEQ ID NOS: 21 and 22 | 17p13.3 | 1905107 | 1905162 |
| BRCA1 | SEQ ID NOS: 23 and 24 | 17q21.31 | 38530811 | 38530871 |
| CASP8 | SEQ ID NOS: 25 and 26 | 2q33.1 | 201830871 | 201830935 |
| RARB | SEQ ID NOS: 27 and 28 | 3p24.2 | 25444559 | 25444621 |
| CDKN2B | SEQ ID NOS: 29 and 30 | 9p21.3 | 21998808 | 21998864 |

[ROC Analysis]

Based on the results for 15 types of genes shown in Table 1 and Figure 1, genes with the results of $P \geq 0.01$ were excluded from target genes. An ROC curve was created regarding methylation in the promoter region of each gene with the result of $P < 0.01$, for which the endpoint was detection of leukoplakia. The area under the curve (AUC) (%) was calculated. The cutoff value to be used for a diagnostic method was set based on sensitivity and specificity obtained from the ROC curve. Table 3 lists those values for 15 types of genes in the descending order for the AUC values.

[Table 3]

| Gene | Cutoff Value | AUC |
|---|---|---|
| RASSF1 | 0.65 | 0.917 |
| DAPK1 | 1.57 | 0.902 |
| CD44 | 1.36 | 0.944 |
| BRCA2 | 0.61 | 0.926 |
| FHIT | 0.59 | 0.899 |
| CDKN2A | 1.48 | 0.819 |
| HIC1 | 1.17 | 0.867 |
| CASP8 | 1.07 | 0.730 |
| RARP | 1.74 | 0.741 |
| CDKN2B | 9.93 | 0.739 |
| CHFR | 0.43 | 0.734 |
| ATM | 1.00 | 0.756 |
| CDKN1B | 0.87 | 0.728 |
| BRCA1 | 0.55 | 0.727 |
| CADM1 | 0.38 | 0.658 |

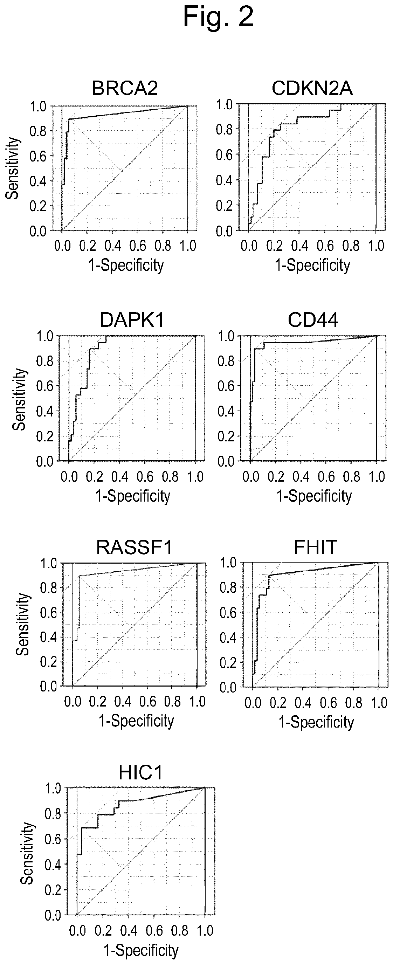

Figure 2 shows ROC curves regarding methylation for DNAs in the promoter regions of 7 genes (RASSF1, DAPK1, CD44, BRCA2, FHIT, CDKN2A, and HIC1), for which the results of AUC > 0.8 were obtained among the genes listed in Table 3. As is apparent from Figure 2, these gene promoter regions were found to be marker candidates more useful for leukoplakia detection.

[Examination of Leukoplakia Detection Accuracy]

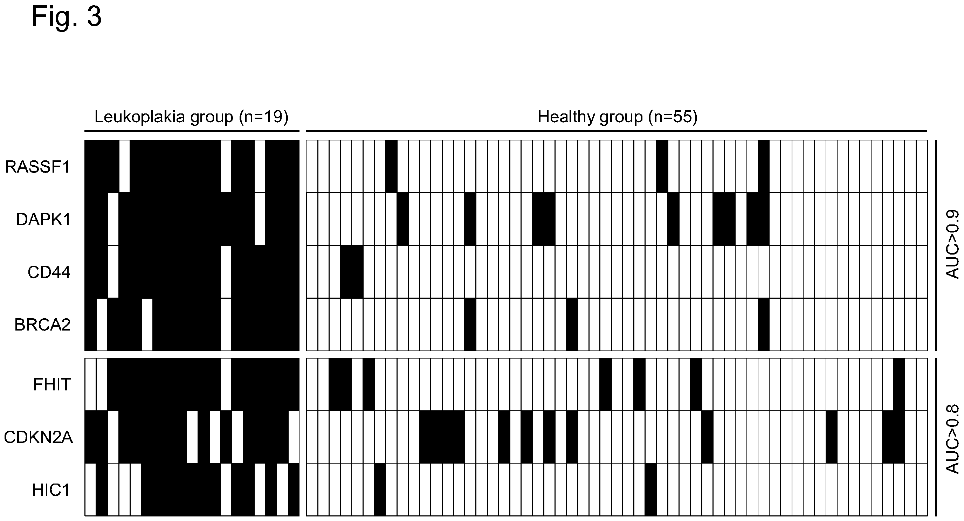

Canonical discriminant analysis of leukoplakia detection was conducted, based on the results of abnormal methylation in the promoter regions of the 7 genes which had favorable AUC values upon ROC analysis. The results are shown in Figure 3. As is apparent from the results, each of DNAs in the promoter regions of the 7 genes was obviously different in terms of tendency of abnormal methylation between the leukoplakia group and the healthy group.

In addition, usefulness of these DNAs as an index for leukoplakia detection was examined using the Fisher's test, sensitivity, and specificity. Table 4 summarizes the occurrence or nonoccurrence of promoter region methylation for each gene.

[Table 4]

Detection accuracy of leukoplakia based on abnormal methylation

| Gene | Patient Group | Unmethylation | Methylation | *P value* | Sensitivity (%) | Specificity (%) |
|---|---|---|---|---|---|---|
| RASSF1 | Healthy group(n=55) | 52 | 3 | <0.001 | 84.2 | 84.2 |
| | Leukoplakia group (n=19) | 3 | 16 | | | |
| CD44 | Healthy group | 53 | 2 | <0.001 | 89.5 | 89.5 |
| | Leukoplakia group | 2 | 17 | | | |
| BRCA2 | Healthy group | 52 | 3 | <0.001 | 84.2 | 84.2 |
| | Leukoplakia group | 3 | 16 | | | |
| DAPK1 | Healthy group | 46 | 9 | <0.001 | 89.5 | 65.4 |
| | Leukoplakia group | 2 | 17 | | | |
| FHIT | Healthy group | 48 | 7 | <0.001 | 84.2 | 69.6 |
| | Leukoplakia group | 3 | 16 | | | |
| HIC1 | Healthy group | 53 | 2 | <0.001 | 63.2 | 85.7 |
| | Leukoplakia group | 7 | 12 | | | |
| CDKN2A | Healthy group | 44 | 11 | <0.001 | 73.7 | 56.0 |
| | Leukoplakia group | 5 | 14 | | | |

As is apparent from the results in Table 4, it was confirmed that DNA in the promoter region of each gene can be significantly distinguished alone between the healthy group and the leukoplakia group. Of these genes, 3 genes: RASSF1, CD44, and BRCA2, each having 80% or higher sensitivity and specificity, were found to be particularly useful.

[Examination of Diagnostic Method based on Combination of Plurality of Genes]

Based on the results for the 3 genes, i.e., RASSF1, CD44, and BRCA2, each of which had favorable detection sensitivity and detection specificity alone, it was examined whether or not a diagnostic method using a combination of detection for methylation in the promoter regions of the genes can be established. As shown in Table 5, it was revealed that abnormal methylation was not detected substantially in the healthy group, while abnormal methylation often occurred in a plurality of genes in the leukoplakia group.

[Table 5]

| Gene Combination | Patient Group | Abnormal methylation | | Sensitivity (%) | Specificity (%) |
| --- | --- | --- | --- | --- | --- |
| | | ≥ 1 | ≥ 2 | | |
| RASFF1+CD44 | Healthy group(n=55) | 55 | 0 | 78.9 | 100 |
| | Leukoplakia group(n=19) | 4 | 15 | | |
| RASFF1+BRCA2 | Healthy group | 54 | 1 | 84.2 | 94.1 |
| | Leukoplakia group | 3 | 16 | | |
| CD44+BRCA2 | Healthy group | 55 | 0 | 78.8 | 100 |
| | Leukoplakia group | 4 | 15 | | |
| RASFF1+CD44+BRCA2 | Healthy group | 54 | 1 | 68.4 | 92.9 |
| | Leukoplakia group | 6 | 13 | | |

Meanwhile, the results in Table 5 indicate that, regarding RASSF1, CD44, and BRCA2, each of which had favorable sensitivity and specificity of 80% or higher alone, specificity tended to increase with any combination of two of them while sensitivity tended to remain at the same level or decrease. This tendency was also confirmed for combination of 3 genes. In the case of analysis using the cutoff value set above, it was found that false negative results could be obtained based on a judgment using a combination of genes, which would not always result in the improvement of accuracy.

[Establishment of Diagnostic Method Based on Combination of Five Genes]

In addition to RASSF1, CD44, and BRCA2, DAPK1 and FHIT, each of which also had favorable detection accuracy alone, were used for conducting canonical discriminant analysis for leukoplakia detection, based on methylation in the promoter regions of these 5 genes as an index.

Logistic analysis was conducted using RASSF1, FHIT, DAPK1, CD44, and BRCA2, and the following formula was obtained:

$$fm = 0.6615648 \times RASSF1 + 0.2922162 \times FHIT + 0.1885340 \times DAPK1 + 0.5206504 \times CD44 + 0.5737340 \times BRCA2.$$

As a result of discrimination using this formula, the integrated evaluation of methylation in the promoter regions of 5 genes represented by the following formula (Fm) varies widely between the leukoplakia group and the healthy group:

$$Fm = -0.02176 + 0.20975 \times (0.6615648 \times RASSF1 + 0.2922162 \times FHIT + 0.1885340 \times DAPK1 + 0.5206504 \times CD44 + 0.5737340 \times BRCA2).$$

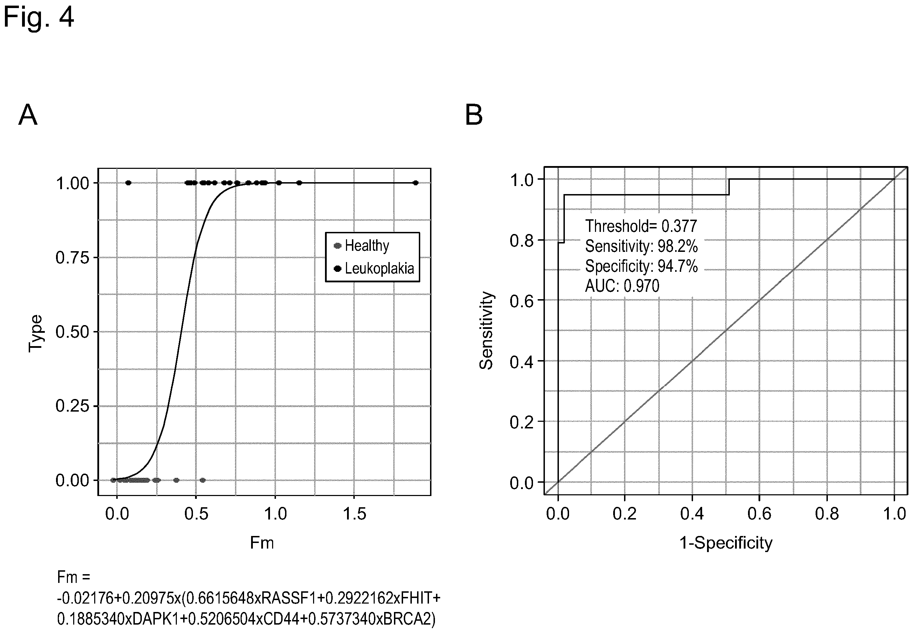

The results are shown in Figure 4. More specifically, there was only one mismatch case in each of the leukoplakia group and the healthy group, and therefore, very high detection accuracy with 98.2% of sensitivity and 94.7% of specificity was achieved.

Industrial Applicability

Since gargled fluid can be noninvasively collected in a convenient manner at low cost, gargled fluid is an ideal test specimen and can be included in evaluation items of general health examination. In addition, as an abnormality in DNA methylation is observed in the early stage of a lesion or before the development of a lesion, it becomes possible to achieve early detection of oral cancer and evaluation of the carcinogenic risk. This allows a subject himself/herself to recognize the carcinogenic risk and contributes to the improvement of lifestyle habits and preventive care.

Claims

1. A method for diagnosis of an oral precancerous lesion in a subj ect by obtaining data by detecting DNA methylation in gargled fluid collected from the subject, wherein the DNA is DNA in a promoter region of one, two or three genes selected from the following group of genes:

   RASSF1 (Ras association domain family member 1, NM_170714.1),
   CD44 (NM_001001391.1), and
   BRCA2 (breast cancer susceptibility gene 2, NM_000059.3).

2. The method according to claim 1, which comprises comparing detected DNA methylation and DNA methylation in a control sample.

3. The method according to claim 1 or 2, wherein DNA methylation in the promoter regions of RASSF1 and BRCA2 genes is detected.

4. The method according to claim 1 or 2, wherein DNA methylation in the promoter regions of DAPK1 (death-associated protein kinase 1, NM_004938.2), and FHIT (fragile histidine triad gene, NM_002012.2) genes is further detected.

5. The method according to claim 1 or 2, wherein DNA methylation is detectable using a probe having any of the nucleotide sequences shown in SEQ ID NOS: 5 to 8, 19, and 20.

6. The method according to any one of claims 1 to 5, wherein the oral precancerous lesion is leukoplakia.

7. Use of primers or at least one probe for detecting abnormal methylation of DNA selected from DNAs of the promoter regions of RASSF1, CD44 and BRCA2 genes, for detecting an oral precancerous lesion in gargled fluid collected from a subject.

8. The use of at least one probe according to claim 7, wherein the at least one probe has any of the nucleotide sequences shown in SEQ ID NOS: 5 to 7, 8, 19, and 20.

9. The use of primers or at least one probe according to claim 7 or 8, wherein the oral precancerous lesion is leukoplakia.


**Patentansprüche**

1. Verfahren für die Diagnose einer präkanzerösen Läsion in der Mundhöhle in einem Subjekt durch Erhalten von Daten durch Erfassen von DNA-Methylierung in gegurgelter Flüssigkeit, die aus dem Subjekt genommen wird, wobei die DNA DNA in einer Promotorregion aus einem, zwei oder drei Genen ist, ausgewählt aus der folgenden Gruppe aus Genen:

   RASSF1 (Ras association domain family member 1, NM_170714.1),
   CD44 (NM_001001391.1) und
   BRCA2 (breast cancer susceptibility gene 2, NM_000059.3).

2. Verfahren nach Anspruch 1, das das Vergleichen von erfasster DNA-Methylierung und DNA-Methylierung in einer Kontrollprobe umfasst.

3. Verfahren nach Anspruch 1 oder 2, wobei DNA-Methylierung in den Promotorregionen von RASSF1- und BRCA2-Genen erfasst wird.

4. Verfahren nach Anspruch 1 oder 2, wobei DNA-Methylierung in den Promotorregionen von DAPK1- (death-associated protein kinase 1, NM 004938.2) und FHIT- (fragile histidine triad gene, NM_002012.2) Genen ferner erfasst wird.

5. Verfahren nach Anspruch 1 oder 2, wobei DNA-Methylierung unter Verwendung einer Sonde mit jeglicher der Nukleotidsequenzen erfasst werden kann, die in SEQ ID NO: 5 bis 8, 19 und 20 gezeigt sind.

**6.** Verfahren nach einem der Ansprüche 1 bis 5, wobei die präkanzeröse Läsion in der Mundhöhle Leukoplakie ist.

**7.** Verwendung von Primern oder von mindestens einer Sonde zum Erfassen von abnormaler Methylierung von DNA, ausgewählt aus DNA der Promotorregionen von RASSF1-, CD44- und BRCA2-Genen, zum Erfassen einer präkanzerösen Läsion in der Mundhöhle in gegurgelter Flüssigkeit, die aus einem Subjekt genommen wird.

**8.** Verwendung von mindestens einer Sonde nach Anspruch 7, wobei die mindestens eine Sonde jegliche der Nukleotidsequenzen hat, die in SEQ ID NO: 5 bis 7, 8, 19 und 20 gezeigt sind.

**9.** Verwendung von Primern oder von mindestens einer Sonde nach Anspruch 7 oder 8, wobei die präkanzeröse Läsion in der Mundhöhle Leukoplakie ist.


**Revendications**

**1.** Procédé de diagnostic d'une lésion précancéreuse buccale chez un sujet par obtention de données par détection de méthylation d'ADN dans un fluide de gargarisme recueilli auprès du sujet, dans lequel l'ADN est de l'ADN dans une région promotrice d'un, deux ou trois gènes choisis dans le groupe de gènes suivant :

RASSF1 (membre de la famille de domaine d'association Ras 1, NM_170714.1),
CD44 (NM_001001391.1), et
BRCA2 (gène de sensibilité au cancer du sein 2, NM_000059.3).

**2.** Procédé selon la revendication 1, qui comprend la comparaison de méthylation d'ADN détectée et de méthylation d'ADN dans un échantillon témoin.

**3.** Procédé selon la revendication 1 ou 2, dans lequel une méthylation d'ADN dans les régions promotrices des gènes RASSF1 et BRCA2 est détectée.

**4.** Procédé selon la revendication 1 ou 2, dans lequel une méthylation d'ADN dans les régions promotrices des gènes DAPK1 (protéine kinase associée à la mort cellulaire 1, NM_004938.2), et FHIT (gène de triade histidine fragile, NM_002012.2) est en outre détectée.

**5.** Procédé selon la revendication 1 ou 2, dans lequel une méthylation d'ADN est détectable à l'aide d'une sonde ayant l'une quelconque des séquences nucléotidiques présentées dans SEQ ID N° : 5 à 8, 19, et 20.

**6.** Procédé selon l'une quelconque des revendications 1 à 5, dans lequel la lésion précancéreuse buccale est la leucoplasie.

**7.** Utilisation d'amorces ou d'au moins une sonde pour détecter une méthylation anormale d'ADN choisi parmi des ADN des régions promotrices des gènes RASSF1, CD44 et BRCA2, pour détecter une lésion précancéreuse buccale dans une fluide de gargarisme recueilli auprès d'un sujet.

**8.** Utilisation d'au moins une sonde selon la revendication 7, dans laquelle l'au moins une sonde a l'une quelconque des séquences nucléotidiques présentées dans SEQ ID N° : 5 à 7, 8, 19, et 20.

**9.** Utilisation d'amorces ou d'au moins une sonde selon la revendication 7 ou 8, dans laquelle la lésion précancéreuse buccale est la leucoplasie.

# Fig. 1

# Fig. 2

# Fig. 3

Leukoplakia group (n=19) — Healthy group (n=55)

RASSF1, DAPK1, CD44, BRCA2 (AUC>0.9)

FHIT, CDKN2A, HIC1 (AUC>0.8)

# Fig. 4

A

Fm =
-0.02176+0.20975x(0.6615648xRASSF1+0.2922162xFHIT+
0.1885340xDAPK1+0.5206504xCD44+0.5737340xBRCA2)

B

Threshold= 0.377
Sensitivity: 98.2%
Specificity: 94.7%
AUC: 0.970

EP 3 346 014 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011229532 A
- JP 2013505428 A

- JP 2015172327 A

**Non-patent literature cited in the description**

- *J. Oral. Maxillofac Surg.,* 06 August 2011, vol. 70 (6), 1486-1494

- *Cancer,* 01 September 2012